(19) Europäisches Patentamt
European Patent Office
Office européen des brevets

(11) Veröffentlichungsnummer: **0 401 582 B1**

# (12) EUROPÄISCHE PATENTSCHRIFT

(45) Veröffentlichungstag der Patentschrift: **20.07.94**

(21) Anmeldenummer: **90109552.1**

(22) Anmeldetag: **19.05.90**

(51) Int. Cl.$^5$: **C07D 471/04**, C07D 498/04, A01N 43/90, //(C07D471/04, 235:00,221:00),(C07D498/04, 263:00,221:00)

## (54) Fünfring-heterocyclisch anellierte Chinolinderivate.

(30) Priorität: **01.06.89 DE 3917883**
**07.02.90 DE 4003587**

(43) Veröffentlichungstag der Anmeldung:
**12.12.90 Patentblatt 90/50**

(45) Bekanntmachung des Hinweises auf die Patenterteilung:
**20.07.94 Patentblatt 94/29**

(84) Benannte Vertragsstaaten:
**AT BE CH DE DK ES FR GB IT LI NL**

(56) Entgegenhaltungen:
**EP-A- 0 090 360**

**CHEMICAL ABSTRACTS Band 66, Nr. 25, 19 Juni 1967, Seite 10745, Zusammenfassung-Nr. 115643s, Columbus, Ohio, US; F.H. CHASE et al.: "The preparation of 2-substituted benzimidazole derivatives containing the ferroin group"**

(73) Patentinhaber: **BASF Aktiengesellschaft**
**Carl-Bosch-Strasse 38**
**D-67063 Ludwigshafen(DE)**

(72) Erfinder: **Brill, Gunter, Dr.**
**Am Schlossergraben 3**
**D-6733 Hassloch(DE)**
Erfinder: **Hagen, Helmut, Dr.**
**Max-Slevogt-Strasse 17 e**
**D-6710 Frankenthal(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruedigerstrasse 13**
**D-6701 Otterstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

JOURNAL OF FLUORINE CHEMISTRY Band 20, 1982, Seiten 573-580; I.G. MOORES et al.: "Alkaline hydrolysis of 2-(trifluoromethyl)imidazo[4,5-f] and -[4,5-h]quinolines"

JOURNAL OF FLUORINE CHEMISTRY Band 41, 1988, Seiten 277-288; I.G. MOORES et al.: "An N-N'-benzyl migration during the formation of some (trifluoromethyl)imidazo[4,5-h]quinolines"

COLLECTION OF THE CZECHOSLOVAK CHEMICAL COMMUNICATIONS Band 52, Nr. 9, September 1987, Seiten 2918-2925; V. MILATA et al.: "Thermal cyclocondensations of 3-N(4- and 5-benzimidazolyl and benztriazolyl)amino derivatives of 2-propenoic acid"

CHEMICAL ABSTRACTS Band 109, Nr. 19, 7. November 1988, Seite 712, Zusammenfassung Nr. 170328z, Columbus, Ohio, US; V. MILATA et al.: "Preparation and spectral properties of imidazo- and trizaloquinolines with angular ring fusion"

CHEMICAL ABSTRACTS Band 107, Nr.25, 21. Dezember 1987, Seite 372, Zusammenfassung Nr. 232001g, Columbus, Ohio, US; F. CHIMENTI et al.: "Studies on amine oxidase-inhibiting substances. Note III. Synthesis and amine oxidase-inhibiting activity of imidiazoquinoline derivatives"

**Beschreibung**

Die Erfindung betrifft Chinolinderivate, verfahren zur ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Substituierte 3H-Imidazo[4,5-h]chinoline und Oxazolo-[5,4h]-chinoline sind beschrieben in J. Fluorine Chem. Bd. 20, S. 573-580 (1982) und Bd. 41, S. 277-288 (1988) sowie in Chemical Abstracts, Band 66, Nr. 25, 1967, Nr. 115 643 s. Substituierte Triazolo[5,4-h]chinoline sind bekannt aus Collect.Czech. Chem. Comm. 52, 2918-2925 (1987) und 53, 1068-1077 (1988). Herbizide Eigenschaften der Chinolinderivate sind dem Stand der Technik nicht zu entnehmen.

Es wurde nun gefunden, daß Fünfring-heterocyclisch anellierte Chinolinderivate der Formel I

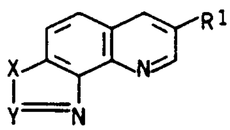

(I),

in der $R^1$, Y und X die folgende Bedeutung haben:

- $R^1$ Wasserstoff, Halogen, Carboxyl oder gegebenenfalls durch $C_1$-$C_4$-Alkoxy, $C_1$-$C_4$-Alkylthio oder Halogen substituiertes $C_1$-$C_6$-Alkyl,
- Y für Stickstoff oder eine Gruppe C-$R^2$, in der
- $R^2$ Wasserstoff, Halogen, Hydroxy, $C_1$-$C_4$-Alkoxy, $C_1$-$C_4$-Alkylthio, einen unsubstituierten oder durch Halogen, Hydroxy, Acetoxy, Cyano, $C_1$-$C_4$-Alkoxy oder $C_1$-$C_4$-Alkylthio substituierten $C_1$-$C_6$-Alkylrest, einen unsubstituierten oder durch $C_1$-$C_4$-Alkyl substituierten $C_3$-$C_6$-Cycloalkylrest, einen unsubstituierten oder durch Nitro, Amino, Halogen, $C_1$-$C_4$-Alkyl, $C_1$-$C_4$-Alkoxy, $C_1$-$C_4$-Alkylthio, $C_1$-$C_4$-Halogenalkyl, Acylamino oder Acyloxy substituierten Benzyl-, Phenethyl-, Phenyl- oder Naphthylrest oder einen gegebenenfalls durch Nitro, $C_1$-$C_4$-Alkyl oder Halogen substituierten 5- oder 6-gliedrigen heterocyclischen Ring mit einem oder zwei Heteroatomen, ausgewählt aus der Gruppe Sauerstoff, Schwefel und Stickstoff bedeutet,
- X Sauerstoff oder den Rest N-$R^3$, wobei $R^3$ Wasserstoff, unsubstituiertes oder durch Halogen, Amino, Mono- oder Dialkylamino, Hydroxy, $C_1$-$C_4$-Alkoxy, $C_1$-$C_4$-Acyloxy substituiertes $C_1$-$C_6$-Alkyl, $C_2$-$C_6$-Alkenyl, $C_2$-$C_6$-Alkinyl, gegebenenfalls $C_1$-$C_4$-Alkyl substituiertes $C_3$-$C_6$-Cycloalkyl, unsubstituiertes oder durch Halogen, $C_1$-$C_4$-Alkyl, $C_1$-$C_4$-Halogenalkyl oder $C_1$-$C_4$-Alkoxy substituiertes Benzyl bedeutet,

herbizide Wirkung haben und gegenüber Kulturpflanzen selektiv sind.

Neben der Verwendung der oben genannten Chinolinderivate I zur Bekämpfung unerwünschten Pflanzenwuchses sind auch die neuen Chinolinderivate I, die folgende Maßgaben enthalten:

a) wenn Y für eine Gruppe C-$R^2$ und $R^1$ für Wasserstoff stehen, $R^2$ nicht Wasserstoff, Methyl, Trifluormethyl, Phenyl, Pyridyl und Benzyl bedeutet, wenn gleichzeitig X für NH steht,

b) $R^2$ nicht Wasserstoff, Methyl oder Trifluormethyl bedeutet, wenn gleichzeitig X für N-Benzyl oder N-Butyl steht; und ferner

c) im Fall von $X = NR^3$ und $Y = N$ die Reste $R^1$ und $R^3$ nicht gleichzeitig Wasserstoff darstellen,

Gegenstand der Erfindung. Besonders bevorzugt sind fünfring-heterocyclisch anellierte Chinolinderivate der Formel I, in der $R^1$ für halogensubstituiertes $C_1$-$C_4$-Alkyl oder Halogen und Y für C-$R^2$ steht.

Die $C_1$-$C_6$-Alkylreste für $R^1$, $R^2$ und $R^3$ in Formel I können unverzweigt oder verzweigt sein und beispielsweise Methyl, Ethyl, n-Propyl, i-Propyl, n-Butyl, i-Butyl, s-Butyl, n-Pentyl oder n-Hexyl bedeuten. Entsprechendes gilt für Alkoxy-, Alkylthio-, Halogenalkyl-, Alkylamino-, Hydroxyalkyl-, Alkoxyalkyl- und Acyloxyalkylreste. Geeignete Substituenten der Alkylreste für $R^1$ und $R^2$ sind Halogen, wie Chlor, Brom oder Fluor, Hydroxy, wie Hydroxymethyl oder 1-Hydroxyethyl, $C_1$-$C_4$-Alkoxy oder $C_1$-$C_4$-Alkylthio, wie Methoxy, Ethoxy, Methylthio oder Ethylthio. Beispiele für solche Reste sind Trichlormethyl, Difluormethyl, Trifluormethyl, Chlormethyl, Methoxymethyl, 1-Methoxyethyl, Methylthiomethyl, Ethylthiomethyl. Bevorzugt sind Reste mit 1 bis 4 Kohlenstoffatomen.

Halogen in Substituenten der Formel I bedeutet beispielsweise Fluor, Chlor oder Brom.

Beispiele für $C_3$-$C_6$-Cycloalkylreste für $R^2$ und $R^3$ sind Cyclopropyl, Cyclobutyl, Cyclopentyl und Cyclohexyl. Die Reste für $R^2$ können zusätzlich durch $C_1$-$C_4$-Alkyl, insbesondere Methyl oder Ethyl, substituiert sein.

Die Benzyl-, 1-Phenethyl-, 2-Phenethyl-, Phenyl-, 1-Naphthyl- und 2-Naphthylreste für $R^2$ können einfach oder mehrfach substituiert sein. Beispiele für Substituenten sind Nitro, Amino, Halogen, wie Fluor, Chlor, Brom, $C_1$-$C_4$-Alkyl, vorzugsweise Methoxy oder Ethoxy, $C_1$-$C_4$-Alkylthio, vorzugsweise Methylthio, $C_1$-$C_4$-Halogenalkyl, wie Trifluormethyl, Acylamino mit 1 bis 4 C-Atomen in der Acylgruppe, beispielsweise Formyl, Acetyl, Propionyl, n-Butyryl oder i-Butyryl und Acyloxy mit 2 bis 5 C-Atomen in der Acylgruppe, beispielsweise Acetyloxy, Propionyloxy, n-Butyryloxy oder i-Butyryloxy.

Beispiele für heterocyclische Reste für $R^2$ sind Pyrrolyl, Furyl, Thienyl, Thiazolyl, Isothiazolyl, Oxazolyl, Isooxazolyl, Pyrazolyl, Imidazolyl, Pyridyl, Pyrimidyl, Pyrazinyl, Pyridazinyl. Diese Reste können durch Nitro, $C_1$-$C_4$-Alkyl, vorzugsweise Methyl oder Ethyl, oder durch Halogen, wie Chlor, Brom oder Fluor, substituiert sein.

$R^3$ im Rest -$NR^3$ steht für Wasserstoff, $C_1$-$C_6$-Alkyl, vorzugsweise $C_1$-$C_4$-Alkyl, insbesondere Methyl oder Ethyl, $C_2$-$C_6$-Alkenyl, insbesondere Vinyl oder Allyl, $C_2$-$C_6$-Alkinyl, insbesondere Propargyl, einen gegebenenfalls durch Halogen, Amino, $C_1$-$C_4$-Mono- oder $C_1$-$C_4$-Dialkylamino, Hydroxy-, $C_1$-$C_4$-Alkoxy- oder $C_1$-$C_4$-Acyloxy substituierten $C_1$-$C_4$-Alkylrest, beispielsweise 2-Chlorethyl, 3-Chlor-n-propyl, 2-Aminoethyl, 2-Methylaminoethyl, 2-Dimethylaminoethyl, 2-Hydroxyethyl, 3-Acetoxypropyl oder 2-Methoxypropyl oder einen gegebenenfalls durch Halogen, $C_1$-$C_4$-Alkyl, $C_1$-$C_4$-Halogenalkyl oder $C_1$-$C_4$-Alkoxy substituierten Benzylrest bedeuten, beispielsweise 4-Chlor-benzyl, 4-Trifluormethyl-benzyl, 4-Methyl-benzyl, 4-Methoxy-benzyl oder 2,4-Dichlor-benzyl.

Man erhält die 3H-Imidazo[4,5-h]chinoline der Formel I durch Umsetzung von 7,8-Diamino-chinolinen der Formel

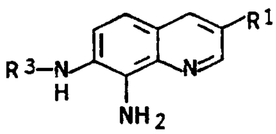

(II)

in an sich bekannter Weise mit Carbonsäuren der Formel $R^2$-COOH (III) oder Orthoestern der Formel $R^2$-C-$(OR^4)_3$ (IV), in der $R^4$ $C_1$-$C_4$-Alkyl bedeutet.

Die Umsetzung verläuft sehr gut bei einer Temperatur im Bereich von 80 bis 120 °C in Anwesenheit von Polyphosphorsäure, bei 80 bis 95 °C in Salzsäure unterschiedlicher Konzentration oder bei 70 bis 100 °C unter Wirkung von 3 bis 6 Äquivalenten IV. Die Menge an III beträgt zweckmäßigerweise 1,1 Mol, bezogen auf 1 Mol II. Größere Überschüsse an III stören nicht.

Die Oxazolo[5,4-h]chinoline der Formel I erhält man entsprechend den 3H-Imidazo[4,5-h]chinolinen durch Umsetzung von 7-Hydroxy-8-aminochinolinen der Formel

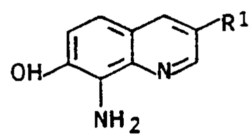

(V)

mit Carbonsäuren der Formel III oder Orthoestern der Formel IV. Die für die Synthese der 3H-Imidazo[4,5-h]chinoline angegebenen Reaktionsbedingungen gelten entsprechend.

Die Triazolo[5,4-h]-chinoline der Formel I erhält man durch Diazotierung von 7,8-Diaminochinolinen der Formel

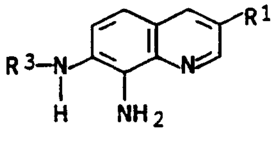

(II)

in an sich bekannter Weise.

Die Umsetzung läuft bei einer Temperatur im Bereich von (-10) bis (+10) °C in Anwesenheit einer Säure, bevorzugt Schwefelsäure oder Salzsäure.

Die Diaminochinoline II bzw. die Aminohydroxychinoline V können, ausgehend von 7-Chlorchinolinen der Formel VI, in der $R^1$ die für Formel I genannten Bedeutungen hat, leicht erhalten werden. Die Synthese

verläuft in an sich bekannter Weise gemäß folgendem Reaktionsschema:

a)

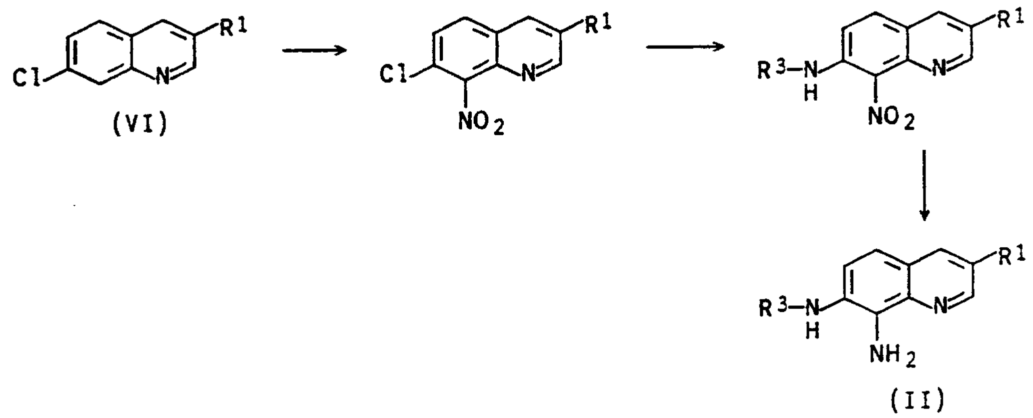

(VI) (II)

b)

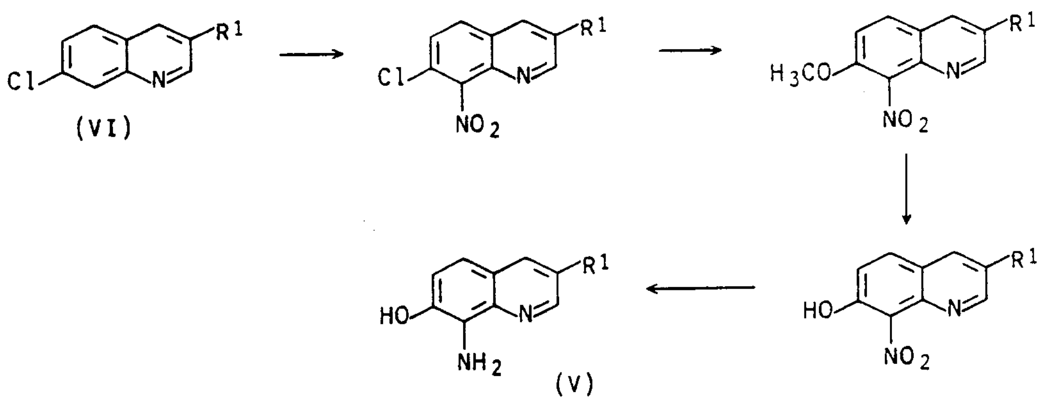

(VI) (V)

Synthesebeispiele

Beispiel 1

3-Methyl-7-chlor-3H-imidazo[4,5-h]chinolin

24,9 g (0,12 mol) 8-Amino-3-chlor-7-methylaminochinolin werden mit 107 g (0,72 mol) Triethylorthoformiat versetzt und 30 min auf 100 ° C erhitzt. Man läßt anschließend successiv auf 0 ° C abkühlen und saugt den Niederschlag (32 g) ab. Die Mutterlauge wird im Vakuum eingeengt und der Rückstand aus einer Essigester/Petrolether-Mischung umkristallisiert. Die so erhaltenen vereinten Feststoffe sind identisch.
Ausbeute: 48,5 g (82 %); Fp.: 188 bis 189 ° C.

Beispiel 2

2-Methyl-3-ethyl-7-chlor-3H-imidazo[4,5-h]chinolin

14 g (64 mmol) 8-Amino-3-chlor-7-ethylaminochinolin werden mit 42 g (0,26 mol) Triethylorthoacetat versetzt und 1 Stunde bei 100 ° C gerührt. Man läßt auf Raumtemperatur kommen, evaporiert überschüssigen Orthoester und kristallisiert den Rückstand aus Essigester um.
Ausbeute: 7,7 g (49 %); Fp.: 145 bis 148 ° C.

Beispiel 3

2-(4-Chlorphenyl)-3-ethyl-7-chlor-3H-imidazo[4,5-h]chinolin

Ein Gemisch bestehend aus 4,1 g (20 mmol) 8-Amino-3-chlor-7-ethylaminochinolin, 3,1 g (20 mmol) 4-Chlorbenzoesäure und 20 ml Polyphosphorsäure wird 1 Stunde auf 120 °C erhitzt. Man läßt auf 90 °C abkühlen und gibt langsam 100 g Eis in die Lösung. Danach gießt man auf 200 g Eis und stellt mit konzentrierter Natronlauge einen pH-Wert von 12 ein. Man saugt den Niederschlag ab, wäscht gründlich mit warmem Wasser nach, trocknet bei 70 °C im Vakuum und kristallisiert aus Essigester um.
Ausbeute: 5,5 g (80 %); Fp.: 110 bis 112 °C.

Beispiel 4

2,7-Dimethyloxazolo[5,4-h]chinolin

3,48 g (20 mmol) 8-Amino-7-hydroxy-3-methylchinolin werden mit 16,2 g (100 mmol) ortho-Essigsäuretriethylester versetzt und 1 Stunde auf 100 °C erwärmt. Man läßt anschließend auf Raumtemperatur abkühlen, versetzt das Reaktionsgemisch unter gutem Rühren mit 50 ml Petrolether und saugt den Niederschlag ab.
Ausbeute: 3,05 g (77 %); Schmp.: 132 bis 134 °C.

Beispiel 5

2-(4-Chlorphenyl)-7-chloroxazolo[5,4-h]chinolin

Ein Gemisch bestehend aus 3,89 g (20 mmol) 8-Amino-3-chlor-7-hydroxychinolin, 3,44 g (22 mmol) 4-Chlorbenzoesäure und 20 ml Polyphosphorsäure wird 2 Stunden auf 120 °C erhitzt. Man läßt auf 90 °C abkühlen, addiert vorsichtig 100 g Eis und 500 ml Wasser. Die so erhaltene Suspension wird mit konzentrierter Natronlauge auf pH 12 gestellt, der Niederschlag wird abgesaugt, mit Wasser gründlich salzfrei gewaschen und im Vakuum bei 60 °C getrocknet. Nach dem Umkristallisieren aus Essigester verbleiben 3,2 g (51 %); Schmp.: >250 °C.

Beispiel 6

3,7-Dimethyl-2-trifluormethyl-3H-imidazo[4,5-h]chinolin

Ein Gemisch bestehend aus 5,61 g (30 mmol) 8-Amino-3-methyl-7-methylaminochinolin und 3,02 ml (40 mmol) Trifluoressigsäure in 40 ml einer 4N Salzsäure wird 2 h auf 95 °C erwärmt. Man läßt anschließend auf Raumtemperatur abkühlen, gießt auf 250 g Eis, stellt mit konz. Natronlauge auf pH 9 und saugt den Niederschlag ab. Man trocknet das Rohprodukt und kristallisiert aus Essigester/Petrolether um.
Ausbeute: 4,8 g (63 %); Schmp.: 233 - 235 °C.

Beispiel 7

3-Ethyl-2-hydroxyimidazo[4,5-h]chinolin

22,4 g (0,12 mol) 8-Amino-7-ethylaminochinolin werden mit 9 g (0,15 Mol) Harnstoff vermischt und unter gutem Rühren 2 h auf 160 °C erhitzt. Man läßt auf 140 °C abkühlen und tropft langsam 50 ml Wasser hinzu. Nach weiterem Abkühlen auf 100 °C addiert man nochmals 100 ml Wasser. Der Niederschlag wird abgesaugt, nochmals mit heißem Wasser nachgewaschen und im Vakuum getrocknet.
Ausbeute: 21,6 g (85 %), Schmp.: > 250 °C.

Beispiel 8

2,7-Dichlor-3-methylimidazo[4,5-h]chinolin

7 g (30 mmol) 7-Chlor-2-hydroxy-3-methylimidazo[4,5-h]chinolin werden mit 7 g (33,6 mmol) Phosphorpentachlorid und 35 ml (0,38 mol) Phosphoroxytrichlorid versetzt. Man refluxiert das Gemisch 15 h, läßt

danach auf Raumtemperatur abkühlen, gießt auf 500 g Eis, stellt mit konz. Natronlauge einen neutralen pH-Wert ein und saugt vom ausgefallenen Niederschlag ab. Nach dem Trocknen und Umkristallieren aus Ethanol erhält man 2,8 g (37 %)
Produkt; Schmp.: 211 bis 213 ° C.

Die in den Synthesebeispielen wiedergegebenen Vorschriften eignen sich unter entsprechender Abwandlung der Ausgangsverbindungen zur Gewinnung weiterer Verbindungen der Formel I. Beispiele für solche Verbindungen sind in den folgenden Tabellen aufgeführt. Sie lassen aufgrund ihrer nahen strukturellen Beziehungen zu den in den Synthesebeispielen genannten Verbindungen eine gleichartige Wirkung erwarten.

Tabelle 1

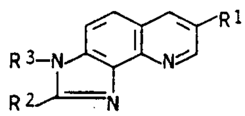

| Nr. | $R^1$ | $R^2$ | $R^3$ | Fp [°C] |
|---|---|---|---|---|
| 9 | H | H | $CH_3$ | 135 - 137 |
| 10 | H | $CH_3$ | $CH_3$ | 238 - 239 |
| 11 | H | $CH(CH_3)_2$ | $CH_3$ | 173 - 174 |
| 12 | H | Cyclopropyl | $CH_3$ | - |
| 13 | H | 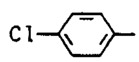 | $CH_3$ | 209 - 211 |
| 14 | H | 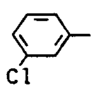 | $CH_3$ | - |
| 15 | H | 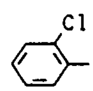 | $CH_3$ | 205 - 207 |
| 16 | H | $CH_2$–$OCH_3$ | $CH_3$ | 112 - 114 |
| 17 | H | $CH_2$–$SCH_3$ | $CH_3$ | 153 - 155 |
| 18 | H | $CH(CH_3)OCH_3$ | $CH_3$ | 78 - 80 |
| 19 | H | $CH_2Cl$ | $CH_3$ | 78 - 80 |
| 20 | H | $CHF_2$ | $CH_3$ | 199 - 201 |
| 21 | H | $CF_3$ | $CH_3$ | 182 - 184 |
| 22 | H | OH | $CH_3$ | > 250 |
| 23 | H | Cl | $CH_3$ | - |
| 24 | H | $CH_3$ | $C_2H_5$ | 150 - 153 |
| 25 | H | $CH(CH_3)_2$ | $C_2H_5$ | 58 - 61 |
| 26 | H | $CH_3OCH_2$ | $C_2H_5$ | 80 - 82 |

| Nr. | $R^1$ | $R^2$ | $R^3$ | Fp [°C] |
|---|---|---|---|---|
| 27 | H | $CF_3$ | $C_2H_5$ | 174 - 177 |
| 7 | H | OH | $C_2H_5$ | > 250 |
| 28 | H | Cl | $C_2H_5$ | - |
| 29 | H | $CH_3$ | $CH_2CH_2CH_3$ | 109 - 111 |
| 30 | H | $CH_3OCH_2$ | $CH_2CH_2CH_3$ | Öl |
| 31 | H | $CF_3$ | $CH_2CH_2CH_3$ | 190 - 192 |
| 32 | H | $CH_3$ | $CH_2CH(CH_3)_2$ | 105 - 108 |
| 33 | H | $CHF_2$ | $CH_2CH(CH_3)_2$ | 145 - 146 |
| 34 | H | $CF_3$ | $CH_2CH(CH_3)_2$ | 210 - 212 |
| 35 | H | $CH_3$ | Cyclopropyl | 224 - 226 |
| 36 | H | $CF_3$ | Cyclopropyl | - |
| 37 | H | $CF_3$ | $CH{=}CH{-}CH_3$ | 172 - 174 |
| 38 | H | $CH_2Cl$ | $CH_2CH_2OH$ | >250 |
| 39 | H | $CH_2{-}OCH_3$ | $CH_2CH_2OH$ | 163 - 165 |
| 40 | H | $CF_3$ | $CH_2CH_2Cl$ | 192 - 194 |
| 41 | H | $CF_3$ | $CH_2CH_2CH_2OH$ | 132 - 135 |
| 42 | H | $CF_3$ | $CH_2CH_2CH_2Cl$ | 173 - 175 |
| 43 | H | $CF_3$ | $CH_2CH(CH_3)OH$ | 208 - 210 |
| 44 | H | $CF_3$ | $CH_2CH(CH_3)Cl$ | 210 - 212 |
| 45 | H | $CH_2Cl$ | $CH_2CH(CH_3)OH$ | >250 |
| 46 | H | $CH_2OCH_3$ | $CH_2CH(CH_3)OH$ | 143 - 145 |
| 47 | $CH_3$ | H | H | - |
| 48 | $CH_3$ | $CH_3$ | H | - |

| Nr. | $R^1$ | $R^2$ | $R^3$ | Fp [°C] |
|---|---|---|---|---|
| 49 | $CH_3$ | $CH(CH_3)_2$ | H | 92 - 95 |
| 50 | $CH_3$ | 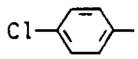 | H | - |
| 51 | $CH_3$ | 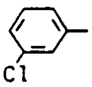 | H | 152 - 153 |
| 52 | $CH_3$ | 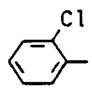 | H | - |
| 53 | $CH_3$ | $CH_3$ | $CH_3$ | 173 - 175 |
| 6 | $CH_3$ | $CF_3$ | $CH_3$ | 233 - 235 |
| 54 | $CH_3$ | $CH_3$ | $C_2H_5$ | 213 - 216 |
| 55 | $CH_3$ | $CH(CH_3)_2$ | $C_2H_5$ | 132 - 133 |
| 56 | $C_2H_5$ | $CH_3$ | $CH_3$ | 114 - 118 |
| 57 | $C_2H_5$ | $CF_3$ | $CH_3$ | 159 - 161 |
| 58 | $CH(CH_3)_2$ | $CF_3$ | $CH_3$ | 149 - 150 |
| 59 | Cl | H | H | - |
| 60 | Cl | $CH_3$ | H | 273 - 275 |
| 61 | Cl | $CH(CH_3)_2$ | H | 215 |
| 62 | Cl | 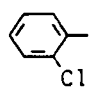 | H | 180 - 182 |
| 63 | Cl | 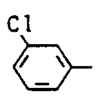 | H | 118 - 121 |
| 64 | Cl | 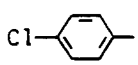 | H | 116 - 119 |
| 65 | Cl | $CF_3$ | H | 234 - 239 |

| Nr. | $R^1$ | $R^2$ | $R^3$ | Fp [°C] |
|---|---|---|---|---|
| 1 | Cl | H | $CH_3$ | 188 - 189 |
| 66 | Cl | $CH_3$ | $CH_3$ | 167 |
| 67 | Cl | $C_2H_5$ | $CH_3$ | 98 - 101 |
| 68 | Cl | $CH_2CH_2CH_3$ | $CH_3$ | 129 |
| 69 | Cl | $CH(CH_3)_2$ | $CH_3$ | 142 - 144 |
| 70 | Cl | $CH_2CH(CH_3)_2$ | $CH_3$ | 113 |
| 71 | Cl | $CH(CH_3)C_2H_5$ | $CH_3$ | 126 - 128 |
| 72 | Cl | $(CH_2)_5CH_3$ | $CH_3$ | 128 |
| 73 | Cl | Cyclopropyl | $CH_3$ | 48 - 50 |
| 74 | Cl | Cyclobutyl | $CH_3$ | 143 - 145 |
| 75 | Cl | 2-Methylcyclopropyl | $CH_3$ | 174 - 177 |
| 76 | Cl | Cyclopentyl | $CH_3$ | 142 - 144 |
| 77 | Cl | Benzyl | $CH_3$ | 194 - 196 |
| 78 | Cl | 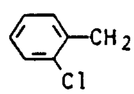 | $CH_3$ | 209 - 211 |
| 79 | Cl | 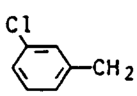 | $CH_3$ | 161 - 163 |
| 80 | Cl | 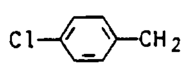 | $CH_3$ | 170 - 173 |
| 81 | Cl | 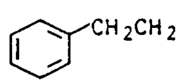 | $CH_3$ | 170 - 173 |
| 82 | Cl | 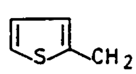 | $CH_3$ | - |

| Nr. | R1 | R2 | R3 | Fp [°C] |
|---|---|---|---|---|
| 83 | Cl | 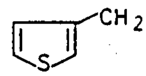 | $CH_3$ | - |
| 84 | Cl | 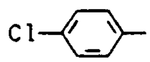 | $CH_3$ | 170 - 172 |
| 85 | Cl | 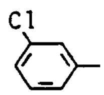 | $CH_3$ | 165 - 168 |
| 86 | Cl | 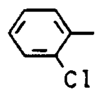 | $CH_3$ | 231 - 232 |
| 87 | Cl | 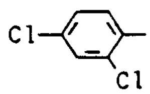 | $CH_3$ | > 250 |
| 88 | Cl | 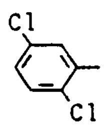 | $CH_3$ | 238 - 240 |
| 89 | Cl | 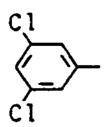 | $CH_3$ | 170 - 172 |
| 90 | Cl | 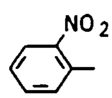 | $CH_3$ | - |
| 91 | Cl | 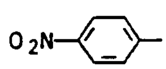 | $CH_3$ | - |
| 92 | Cl | 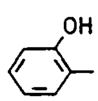 | $CH_3$ | - |
| 93 | Cl | 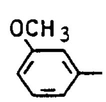 | $CH_3$ | 177 - 179 |

| Nr. | $R^1$ | $R^2$ | $R^3$ | Fp [°C] |
|---|---|---|---|---|
| 94 | Cl | 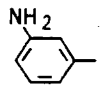 | $CH_3$ | 216 - 217 |
| 95 | Cl | 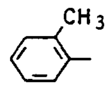 | $CH_3$ | - |
| 96 | Cl | 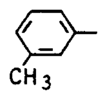 | $CH_3$ | 196 - 198 |
| 97 | Cl | 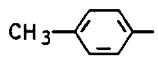 | $CH_3$ | 190 - 191 |
| 98 | Cl | 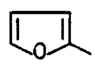 | $CH_3$ | - |
| 99 | Cl | 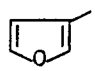 | $CH_3$ | 165 |
| 100 | Cl | 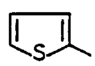 | $CH_3$ | 95 - 96 |
| 101 | Cl | 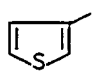 | $CH_3$ | 204 - 205 |
| 102 | Cl | $CH_2OCH_3$ | $CH_3$ | 138 - 140 |
| 103 | Cl | $CH_2OC_6H_5$ | $CH_3$ | 237 - 240 |
| 104 | Cl | $CH_2SCH_3$ | $CH_3$ | 145 - 148 |
| 105 | Cl | $CH_2Cl$ | $CH_3$ | > 250 |
| 106 | Cl | $CH_2CN$ | $CH_3$ | - |
| 107 | Cl | $CH_2O\overset{O}{\overset{\|}{C}}CH_3$ | $CH_3$ | - |
| 108 | Cl | $CH(CH_3)OH$ | $CH_3$ | 65 - 66 |

| Nr. | $R^1$ | $R^2$ | $R^3$ | Fp [°C] |
|---|---|---|---|---|
| 109 | Cl | $CH(CH_3)OCH_3$ | $CH_3$ | 132 - 135 |
| 110 | Cl | $CHF_2$ | $CH_3$ | 215 - 216 |
| 111 | Cl | $CHCl_2$ | $CH_3$ | 110 - 115 |
| 112 | Cl | $CF_3$ | $CH_3$ | 173 - 175 |
| 113 | Cl | $CCl_3$ | $CH_3$ | 247 - 250 |
| 114 | Cl | $CONH_2$ | $CH_3$ | > 250 |
| 115 | Cl | SH | $CH_3$ | > 250 |
| 116 | Cl | $SCH_3$ | $CH_3$ | 140 - 141 |
| 8 | Cl | Cl | $CH_3$ | 210 - 213 |
| 117 | Cl | $OCH_3$ | $CH_3$ | - |
| 118 | Cl | $OC_2H_5$ | $CH_3$ | 125 - 128 |
| 119 | Cl | $CF_3$ | $CH=CH_2$ | 125 - 128 |
| 2 | Cl | H | $C_2H_5$ | 215 - 217 |
| 120 | Cl | $CH_3$ | $C_2H_5$ | 145 - 148 |
| 121 | Cl | $C_2H_5$ | $C_2H_5$ | 138 - 140 |
| 122 | Cl | $CH(CH_3)_2$ | $C_2H_5$ | 127 - 130 |
| 123 | Cl | Cyclopropyl | $C_2H_5$ | 146 - 148 |
| 124 | Cl | $CH_2Cl$ | $C_2H_5$ | > 250 |
| 125 | Cl | $CH_2OCH_3$ | $C_2H_5$ | 72 - 74 |
| 126 | Cl | $CF_3$ | $C_2H_5$ | 140 - 143 |
| 127 | Cl | OH | $C_2H_5$ | > 250 |
| 128 | Cl | Cl | $C_2H_5$ | - |

| Nr. | $R^1$ | $R^2$ | $R^3$ | Fp [°C] |
|---|---|---|---|---|
| 129 | Cl | 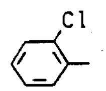 | $C_2H_5$ | 198 |
| 130 | Cl | 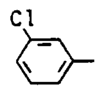 | $C_2H_5$ | 155 - 156 |
| 3 | Cl | 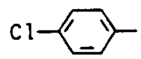 | $C_2H_5$ | 110 - 112 |
| 131 | Cl | 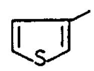 | $C_2H_5$ | 112 - 114 |
| 132 | Cl | H | $CH_2CH_2CH_3$ | 96 - 98 |
| 133 | Cl | $CH_3$ | $CH_2CH_2CH_3$ | 128 - 130 |
| 134 | Cl | $CH_2OCH_3$ | $CH_2CH_2CH_3$ | 142 - 144 |
| 135 | Cl | $CF_3$ | $CH_2CH_2CH_3$ | 178 - 181 |
| 136 | Cl | $CH_3$ | $CH_2{-}CH{=}CH_2$ | 138 - 140 |
| 137 | Cl | $CH_3$ | $CH{=}CH{-}CH_3$ | 234 - 236 |
| 138 | Cl | H | $CH_2CH(CH_3)_2$ | 111 - 113 |
| 139 | Cl | $CH_3$ | $CH_2CH(CH_3)_2$ | 123 - 125 |
| 140 | Cl | $CF_3$ | $CH_2CH(CH_3)_2$ | 152 - 155 |
| 141 | Cl | H | Benzyl | 163 |
| 142 | Cl | $CH_3$ | Benzyl | - |
| 143 | Cl | H | Cyclopropyl | 130 - 132 |
| 144 | Cl | $CH_3$ | Cyclopropyl | 229 - 231 |
| 145 | Cl | $C_2H_5$ | Cyclopropyl | - |
| 146 | Cl | $CHF_2$ | Cyclopropyl | 180 - 183 |

| Nr. | $R^1$ | $R^2$ | $R^3$ | Fp [°C] |
|---|---|---|---|---|
| 147 | Cl | $CF_3$ | Cyclopropyl | - |
| 148 | Cl | H | Cyclopentyl | - |
| 149 | Cl | $CH_3$ | Cyclopentyl | 167 - 170 |
| 150 | Cl | $CH_3$ | $CH_2CH_2OH$ | 245 - 247 |
| 151 | Cl | $CH_2OCH_3$ | $CH_2CH_2OH$ | > 250 |
| 152 | Cl | $CF_3$ | $CH_2CH_2OH$ | 243 - 245 |
| 153 | Cl | $CONH_2$ | $CH_2CH_2OH$ | > 250 |
| 154 | Cl | OH | $CH_2CH_2OH$ | 227 - 229 |
| 155 | Cl | $CH_3$ | $CH_2CH_2O\overset{O}{\overset{\|}{C}}CH_3$ | Harz |
| 156 | Cl | $CH_2OCH_3$ | $CH_2CH(CH_3)OH$ | 190 - 192 |
| 157 | Cl | $CF_3$ | $CH_2CH(CH_3)OH$ | 197 - 200 |
| 158 | Cl | OH | $CH_2CH(CH_3)OH$ | 232 - 233 |
| 159 | Cl | $CH_3$ | $CH_2CH(CH_3)O\overset{O}{\overset{\|}{C}}CH_3$ | Harz |
| 160 | Cl | $CH_2OCH_3$ | $CH_2CH_2CH_2OH$ | 159 - 162 |
| 161 | Cl | $CF_3$ | $CH_2CH_2CH_2OH$ | 150 - 153 |
| 162 | Cl | OH | $CH_2CH_2CH_2OH$ | > 250 |
| 163 | Cl | $CF_3$ | $CH_2CH_2CH_2O\overset{O}{\overset{\|}{C}}CH_3$ | 149 - 151 |
| 164 | Cl | $CF_3$ | $CH_2CH_2Cl$ | 172 - 174 |
| 165 | Cl | $CF_3$ | $CH_2CH(CH_3)Cl$ | 161 - 162 |
| 166 | Cl | OH | $CH_2CH_2CH_2Cl$ | 213 - 215 |
| 167 | Cl | $CH_2OCH_3$ | $CH_2CH_2N(CH_3)_2$ | 170 - 171 |
| 168 | Cl | $CF_3$ | $CH_2CH_2N(CH_3)_2$ | 166 - 168 |

Tabelle 2

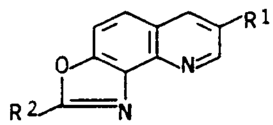

(Ib)

| Nr. | $R^1$ | $R^2$ | Fp [°C] |
|---|---|---|---|
| 169 | H | $CH_3$ | 155 - 156 |
| 170 | H | $C_2H_5$ | 92 - 93 |
| 171 | Cl | H | 138 |
| 172 | Cl | $CH_3$ | 162 - 164 |
| 173 | Cl | 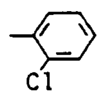 | 164 |
| 174 | Cl | 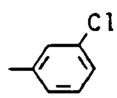 | 232 |
| 5 | Cl | 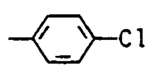 | > 250 |
| 175 | $CH_3$ | H | 115 - 117 |
| 4 | $CH_3$ | $CH_3$ | 132 - 134 |
| 176 | $CH_3$ | $C_2H_5$ | 79 - 80 |
| 177 | $CH_3$ | 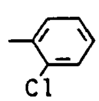 | 144 - 146 |
| 178 | $CH_3$ | 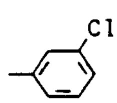 | 130 - 131 |
| 179 | $CH_3$ | 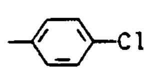 | 243 - 245 |

| Nr. | $R^1$ | $R^2$ | Fp [°C] |
|---|---|---|---|
| 180 | H | H | - |
| 181 | H | $CH_3$ | 191 - 194 |
| 182 | H | $C_2H_5$ | 108 - 110 |
| 183 | H | $n-C_3H_7$ | 109 - 112 |
| 184 | H | $i-C_4H_9$ | 104 - 107 |
| 185 | H | $c-C_3H_5$ | 205 - 208 |
| 186 | $CH_3$ | $CH_3$ | 190 - 192 |
| 187 | $C_2H_5$ | $CH_3$ | 88 - 90 |
| 188 | Cl | H | > 280 |
| 189 | Cl | $CH_3$ | 192 - 195 |
| 190 | Cl | $C_2H_5$ | 141 - 144 |
| 191 | Cl | $n-C_3H_7$ | 145 - 148 |
| 192 | Cl | $c-C_3H_5$ | 103 - 107 |
| 193 | Cl | $i-C_4H_9$ | 145 - 150 |
| 194 | Cl | $HOCH_2CH_2$ | 222 - 224 |
| 195 | Cl | $HOCH(CH_3)-CH_2$ | 202 - 205 |
| 196 | | $HOCH_2CH_2CH_2$ | 124 - 127 |
| 197 | Cl | $ClCH_2CH_2CH_2$ | 134 - 136 |
| 198 | Cl | $AcO-CH_2CH_2$ | 187 - 190 |

Die Fünfring-heterocyclisch anellierten Chinolinderivate I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Napthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z. B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracatat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoff und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 93 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 36 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 37 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 60 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser enthält man eine wäßrige Dispersion, die 0,02 Gew.% des Wirkstoffs enthält.

V. 20 Gewichtsteile des Wirkstoffs Nr. 36 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 37 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 69 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 63 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,001 bis 4, vorzugsweise 0,01 bis 2 kg/ha.

Zur Verbreitung des Wirkungsspektrums und zur Erzielung synergistischer Effekte können die Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenoxy- bzw. Heteroaryloxy-phenoxypropionsäuren sowie deren Salze, Ester und Amide und andere in Betracht.

Außerdem kann es von Nutzen sein, die Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrationen zugesetzt werden.

In Anbetracht der Vielseitigkeit der Applikationsmethoden können die erfindungsgemäßen Verbindungen bzw. sie enthaltende Mittel noch in einer großen Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |

| Botanischer Name | Deutscher Name |
|---|---|
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Risphenhirse |
| Phaseolus lunatus | Mondbohne |
| Phaseolus mungo | Erdbohne |

| Botanischer Name | Deutscher Name |
|---|---|
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Anwendungsbeispiele

Die herbizide Wirkung der 3H-Imidazo[4,5-h](Oxazolo[5,4-h])chinoline der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät.

Zum Zwecke der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 1,0 kg Wirkstoff/ha.

Die Versuchsgefäße werden im Gewächshaus aufgestellt, wobei für wärmeliebende Arten wärmere Bereiche (20 bis 35 ° C) und für solche gemäßigter Klimate 10 bis 20 ° C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet sind nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Cassia tora | - |
| Ipomoea spp. | Prunkwindenarten |
| Lamium amplexicaule | Stengelumfassende Taubnessel |

Bei einer Aufwandmenge von 1,0 kg Wirkstoff/ha lassen sich im Nachauflaufverfahren mit dem Wirkstoff Nr. 1 breitblättrige unerwünschte Pflanzen sehr gut bekämpfen, wobei der Wirkstoff gleichzeitig verträglich ist für die Kulturpflanze Mais.

Die herbizide Wirkung der Triazolo[5,4h]chinoline der Formel I auf das Wachstum der Testpflanzen wird durch folgende Gewächshausversuche gezeigt.

Als Kulturgefäße dienen Plastikblumentöpfe mit lehmigem Sand mit etwa 3,0 % Humus als Substrat. Die Samen der Testpflanzen werden nach Arten getrennt eingesät.

Bei Vorauflaufbehandlung werden die in Wasser suspendierten oder emulgierten Wirkstoffe direkt nach Einsaat mittels fein verteilender Düsen aufgebracht. Anschließend wurden die Gefäße leicht beregnet, um Keimung und Wachstum in Gang zu bringen. Danach wurden die Gefäße mit durchsichtigen Plastikhauben abgedeckt, bis die Pflanzen angewachsen waren. Diese Abdeckung fördert ein gleichmäßiges Keimen der Testpflanzen, sofern dies nicht durch die Wirkstoffe beeinträchtigt wird.

Zum Zweck der Nachauflaufbehandlung werden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Pflanzen wurden artenspezifisch bei Temperaturen von 10 bis 25 °C bzw. 20 bis 35 °C gehalten. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Abkürz. | Lateinischer Name | Deutsche Name | Englischer Name |
|---|---|---|---|
| CHEAL | Chenopodium album | Weißer Gänsefuß | lambsquarters (goosefoot) |
| STEME | Stellaria media | Vogelsternmiere | chickweed |

Mit 2,0 kg/ha a.S. im Nachauflaufverfahren eingesetzt, lassen sich mit den Beispielen 191 und 192 breitblättrige unerwünschte Pflanzen sehr gut bekämpfen.

**Patentansprüche**

**1.** Fünfring-heterocyclisch anellierte Chinolinderivate der Formel I

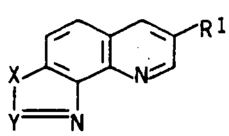

(I)

in der $R^1$, Y und X die folgende Bedeutung haben:

- $R^1$ Wasserstoff, Halogen, Carboxyl oder gegebenenfalls durch $C_1$-$C_4$-Alkoxy, $C_1$-$C_4$-Alkylthio oder Halogen substituiertes $C_1$-$C_6$-Alkyl,
- Y Stickstoff oder eine Gruppe C-$R^2$, in der
- $R^2$ Wasserstoff, Halogen, Hydroxy, $C_1$-$C_4$-Alkoxy, $C_1$-$C_4$-Alkylthio, einen unsubstituierten oder durch Halogen, Hydroxy, Acetoxy, Cyano, $C_1$-$C_4$-Alkoxy oder $C_1$-$C_4$-Alkylthio substituierten $C_1$-$C_6$-Alkylrest, einen unsubstituierten oder durch $C_1$-$C_4$-Alkyl substituierten $C_3$-$C_6$-Cycloalkylrest, einen unsubstituierten oder durch Nitro, Amino, Halogen, $C_1$-$C_4$-Alkyl, $C_1$-$C_4$-Alkoxy, $C_1$-$C_4$-Alkylthio, $C_1$-$C_4$-Halogenalkyl, Acylamino oder Acyloxy substituierten Benzyl-, Phenethyl-, Phenyl-oder Naphthylrest oder einen gegebenenfalls durch Nitro, $C_1$-$C_4$-Alkyl oder Halogen substituierten 5- oder 6-gliedrigen heterocyclischen Ring mit einem oder zwei Heteroatomen, ausgewählt aus der Gruppe Sauerstoff, Schwefel und Stickstoff bedeutet,
- X Sauerstoff oder den Rest N-$R^3$, wobei $R^3$ Wasserstoff, unsubstituiertes oder durch Halogen, Amino, Mono- oder Dialkylamino, Hydroxy, $C_1$-$C_4$-Alkoxy, $C_1$-$C_4$-Alkylacyloxy substituiertes $C_1$-$C_6$-Alkyl, $C_2$-$C_6$-Alkenyl, $C_2$-$C_6$-Alkinyl, unsubstituiertes oder durch $C_1$-$C_4$-Alkyl substituiertes $C_3$-$C_6$-Cycloalkyl, unsubstituiertes oder durch Halogen, $C_1$-$C_4$-Alkyl, $C_1$-$C_4$-Halogenalkyl oder $C_1$-$C_4$-Alkoxy substituiertes Benzyl bedeutet,

mit der Maßgabe, daß wenn Y für eine Gruppe C-$R^2$ und $R^1$ für Wasserstoff stehen, $R^2$ nicht Wasserstoff, Methyl, Trifluormethyl, Phenyl, Pyridyl und Benzyl bedeutet, wenn gleichzeitig X für NH steht sowie $R^2$ nicht Wasserstoff, Methyl oder Trifluormethyl bedeutet, wenn gleichzeitig X für N-Benzyl oder N-Butyl steht; und ferner mit der Maßgabe, daß im Fall von X = N$R^3$ und Y = N die Reste $R^1$ und $R^3$ nicht gleichzeitig Wasserstoff darstellen.

**2.** Fünfring-heterocyclisch anellierte Chinolinderivate der Formel I gemäß Anspruch 1, in der $R^1$ für halogensubstituiertes $C_1$-$C_4$-Alkyl oder Halogen und Y für C-$R^2$ steht.

**3.** Verfahren zur Herstellung von 3H-Imidazo[4,5-h]chinolinen der Formel I gemäß Anspruch 1, in der X für N-$R^3$ und Y für C-$R^2$ stehen, dadurch gekennzeichnet, daß man 7,8-Diamino-chinoline der Formel II

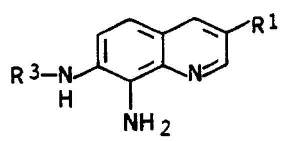

(II)

in an sich bekannter Weise mit Carbonsäuren der Formel $R^2$-COOH (III) oder Orthoestern der Formel $R^2C(OR^4)_3$ (IV), in der $R^4$ $C_1$-$C_4$-Alkyl bedeutet, umsetzt.

**4.** Verfahren zur Herstellung von Oxazolo[5,4-h]chinolinen der Formel I gemäß Anspruch 1, in der X für Sauerstoff und Y für C-$R^2$ stehen, dadurch gekennzeichnet, daß man 7-Hydroxy-8-aminochinoline der Formel

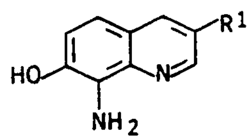

(V)

in an sich bekannter Weise mit Carbonsäuren der Formel $R^2$-COOH (III) oder Orthoestern der Formel $R^2$-$C(OR^4)_3$ (IV), in der $R^4$ $C_1$-$C_4$-Alkyl bedeutet, umsetzt.

**5.** Verfahren zur Herstellung von Triazolo[5,4-h]chinolinen der Formel I gemäß Anspruch 1, in der X für N-$R^3$ und Y für Stickstoff stehen, dadurch gekennzeichnet, daß man 7,8-Diaminochinoline der Formel VI

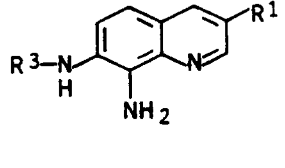

(VI)

in an sich bekannter Weise durch Diazotierung mit Natriumnirit in salzsaurer Lösung cyclisiert.

6. Herbizid, enthaltend inerte Zusatzstoffe und ein Fünfring-heterocyclisch anelliertes Chinolinderivat der Formel I gemäß Anspruch 1.

7. Verfahren zur Bekämpfung unerwünschten Pflanzenwuchses, dadurch gekennzeichnet, daß man die Pflanzen und/oder ihren Standort mit einer herbizid wirksamen Menge eines Fünfring-heterocyclisch anellierten Chinolinderivates der Formel I

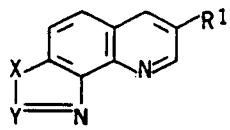

(I)

in der $R^1$, Y und X die folgende Bedeutung haben:

$R^1$ Wasserstoff, Halogen, Carboxyl oder gegebenenfalls durch $C_1$-$C_4$-Alkoxy, $C_1$-$C_4$-Alkylthio oder Halogen substituiertes $C_1$-$C_6$-Alkyl,

Y Stickstoff oder eine Gruppe C-$R^2$, in der

$R^2$ Wasserstoff, Halogen, Hydroxy, $C_1$-$C_4$-Alkoxy, $C_1$-$C_4$-Alkylthio, einen unsubstituierten oder durch Halogen, Hydroxy, Acetoxy, Cyano, $C_1$-$C_4$-Alkoxy oder $C_1$-$C_4$-Alkylthio substituierten $C_1$-$C_6$-Alkylrest, einen unsubstituierten oder durch $C_1$-$C_4$-Alkyl substituierten $C_3$-$C_6$-Cycloalkylrest, einen unsubstituierten oder durch Nitro, Amino, Halogen, $C_1$-$C_4$-Alkyl, $C_1$-$C_4$-Alkoxy, $C_1$-$C_4$-Alkylthio, $C_1$-$C_4$-Halogenalkyl, Acylamino oder Acyloxy substituierten Benzyl-, Phenethyl-, Phenyl-oder Naphthylrest oder einen gegebenenfalls durch Nitro, $C_1$-$C_4$-Alkyl oder Halogen substituierten 5- oder 6-gliedrigen heterocyclischen Ring mit einem oder zwei Heteroatomen, ausgewählt aus der Gruppe Sauerstoff, Schwefel und Stickstoff bedeutet,

X Sauerstoff oder den Rest N-$R^3$, wobei $R^3$ Wasserstoff, unsubstituiertes oder durch Halogen, Amino, Mono- oder Dialkylamino, Hydroxy, $C_1$-$C_4$-Alkoxy, $C_1$-$C_4$-Alkylacyloxy substituiertes $C_1$-$C_6$-Alkyl, $C_2$-$C_6$-Alkenyl, $C_2$-$C_6$-Alkinyl, gegebenenfalls $C_1$-$C_4$-Alkyl substituiertes $C_3$-$C_6$-Cycloalkyl, unsubstituiertes oder durch Halogen, $C_1$-$C_4$-Alkyl, $C_1$-$C_4$-Halogenalkyl oder $C_1$-$C_4$-Alkoxy substituiertes Benzyl bedeutet,

behandelt.

## Claims

1. A quinoline derivative which is fused to a five-membered heterocyclic ring and has the formula I

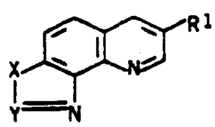

(I)

wherein

$R^1$ is hydrogen, halogen, carboxyl, or $C_1$-$C_6$-alkyl which is unsubstituted or substituted by $C_1$-$C_4$-alkoxy, $C_1$-$C_4$-alkylthio or halogen,

Y is nitrogen or C-$R^2$, where

$R^2$ is hydrogen, halogen, hydroxyl, $C_1$-$C_4$-alkoxy, $C_1$-$C_4$-alkylthio, or $C_1$-$C_6$-alkyl which is unsubstituted or substituted by halogen, hydroxyl, acetoxy, cyano, $C_1$-$C_4$-alkoxy or $C_1$-$C_4$-alkylthio, or is $C_3$-$C_6$-cycloalkyl which is unsubstituted or substituted by $C_1$-$C_4$-alkyl, or is benzyl, phenethyl, phenyl or naphthyl, each of which is unsubstituted or substituted by nitro, amino, halogen, $C_1$-$C_4$-alkyl, $C_1$-$C_4$-alkoxy, $C_1$-$C_4$-alkylthio, $C_1$-$C_4$-haloalkyl, acylamino or acyloxy, or is a 5- or 6-membered heterocyclic ring which has one or two hetero atoms selected from the group consisting of oxygen, sulfur and nitrogen and is unsubstituted or substituted by nitro, $C_1$-$C_4$-alkyl or halogen,

X is oxygen or N-$R^3$, where $R^3$ is hydrogen, $C_1$-$C_6$-alkyl, $C_2$-$C_6$-alkenyl or $C_2$-$C_6$-alkynyl, each of which is unsubstituted or substituted by halogen, amino, mono- or dialkylamino, hydroxyl,

$C_1$-$C_4$-alkoxy or $C_1$-$C_4$-alkylacyloxy, or is $C_3$-$C_6$-cycloalkyl which is unsubstituted or substituted by $C_1$-$C_4$-alkyl or is benzyl which is unsubstituted or substituted by halogen, $C_1$-$C_4$-alkyl, $C_1$-$C_4$-haloalkyl or $C_1$-$C_4$-alkoxy,

with the proviso that $R^2$ is not hydrogen, methyl, trifluoromethyl, phenyl, pyridyl or benzyl when Y is C-$R^2$, $R^1$ is hydrogen and X is NH, and $R^2$ is not hydrogen, methyl or trifluoromethyl when X is N-benzyl or N-butyl; and with the further proviso that $R^1$ and $R^3$ are not both hydrogen when X = $NR^3$ and Y = N.

**2.** A quinoline derivative which is fused to a five-membered heterocyclic ring and has the formula I, as claimed in claim 1, where $R^1$ is halogen-substituted $C_1$-$C_4$-alkyl or halogen and Y is C-$R^2$.

**3.** A process for preparing a 3H-imidazo[4,5-h]quinoline of the formula I as claimed in claim 1, where X is N-$R^3$ and Y is C-$R^2$, which comprises reacting a 7,8-diaminoquinoline of the formula II

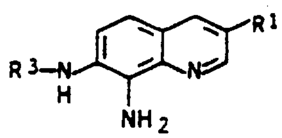

(II)

in a conventional manner with a carboxylic acid of the formula $R^2$-COOH (III) or an orthoester of the formula $R^2C(OR^4)_3$ (IV) where $R^4$ is $C_1$-$C_4$-alkyl.

**4.** A process for preparing an oxazolo[5,4-h]quinoline of the formula I as claimed in claim 1, where X is oxygen and Y is C-$R^2$, which comprises reacting a 7-hydroxy-8-aminoquinoline of the formula

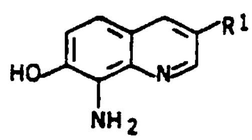

(V)

in a conventional manner with a carboxylic acid of the formula $R^2$-COOH (III) or an orthoester of the formula $R^2$-$C(OR^4)_3$ (IV) where $R^4$ is $C_1$-$C_4$-alkyl.

**5.** A process for preparing a triazolo[5,4-h]quinoline of the formula I as claimed in claim 1, where X is N-$R^3$ and Y is nitrogen, which comprises cyclizing a 7,8-diaminoquinoline of the formula VI

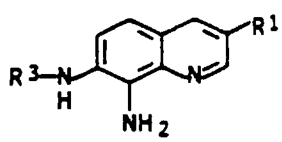

(VI)

in a conventional manner by diazotization with sodium nitrite in hydrochloric acid solution.

**6.** A herbicide comprising inert additives and a quinoline derivative which is fused to a five-membered heterocyclic ring and has the formula I as claimed in claim 1.

**7.** A method for controlling undesired plant growth, wherein the plants and/or their habitat are treated with a herbicidally effective amount of a quinoline derivative which is fused to a five-membered heterocyclic ring and has the formula I

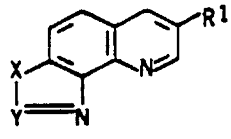

(I)

where

- $R^1$ is hydrogen, halogen, carboxyl, or $C_1$-$C_6$-alkyl which is unsubstituted or substituted by $C_1$-$C_4$-alkoxy, $C_1$-$C_4$-alkylthio or halogen,
- Y is nitrogen or C-$R^2$, where
- $R^2$ is hydrogen, halogen, hydroxyl, $C_1$-$C_4$-alkoxy, $C_1$-$C_4$-alkylthio, or $C_1$-$C_6$-alkyl which is unsubstituted or substituted by halogen, hydroxyl, acetoxy, cyano, $C_1$-$C_4$-alkoxy or $C_1$-$C_4$-alkylthio, or is $C_3$-$C_6$-cycloalkyl which is unsubstituted or substituted by $C_1$-$C_4$-alkyl or is benzyl, phenethyl, phenyl or naphthyl, each of which is unsubstituted or substituted by nitro, amino, halogen, $C_1$-$C_4$-alkyl, $C_1$-$C_4$-alkoxy, $C_1$-$C_4$-alkylthio, $C_1$-$C_4$-haloalkyl, acylamino or acyloxy, or is a 5- or 6-membered heterocyclic ring which has one or two hetero atoms selected from the group consisting of oxygen, sulfur and nitrogen and is unsubstituted or substituted by nitro, $C_1$-$C_4$-alkyl or halogen,
- X is oxygen or N-$R^3$, where $R^3$ is hydrogen, $C_1$-$C_6$-alkyl, $C_2$-$C_6$-alkenyl or $C_2$-$C_6$-alkynyl, each of which is unsubstituted or substituted by halogen, amino, mono- or dialkylamino, hydroxyl, $C_1$-$C_4$-alkoxy or $C_1$-$C_4$-alkylacyloxy, or is $C_3$-$C_6$-cycloalkyl which is unsubstituted or substituted by $C_1$-$C_4$-alkyl or is benzyl which is unsubstituted or substituted by halogen, $C_1$-$C_4$-alkyl, $C_1$-$C_4$-haloalkyl or $C_1$-$C_4$-alkoxy.

**Revendications**

**1.** Dérivés de quinoléine condensés hétérocyclique à noyau pentagonal de formule I

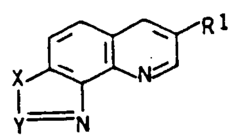

(I)

dans laquelle $R^1$, Y et X ont les significations suivantes :

- $R^1$ hydrogène, halogène, carboxyle ou alkyle en C1-C6 éventuellement substitué par alcoxy en C1-C4, alkylthio en C1-C4 ou halogène
- Y azote ou un groupe C-$R^2$, dans lequel
- $R^2$ représente hydrogène, halogène, hydroxy, alcoxy en C1-C4, alkylthio en C1-C4, un reste alkyle en C1-C6 non substitué ou substitué par halogène, hydroxy, alcoxy, cyano, alcoxy en C1-C4 ou alkylthio en C1-C4, un reste cycloalkyle en C3-C6 non substitué ou substitué par alkyle en C1-C4, un reste benzyle, phénéthyle, phényle ou naphtyle non substitue ou substitué par nitro, amino, halogène, alkyle en C1-C4, alcoxy en C1-C4, alkyle en C1-C4-thio, halogénalkyle en C1-C4, acylamino ou acyloxy, ou un noyau hétérocyclique à 5 ou 6 chaînons, avec un ou deux hétéroatomes choisis dans le groupe oxygène soufre et azote, noyau éventuellement substitué par nitro, alkyle en C1-C4 ou halogène
- X oxygène ou le reste N-$R^3$, $R^3$ représentant hydrogène, alkyle en C1-C6, alcényle en C2-C6, alcinyle en C2-C6 non substitué ou substitué par halogène, amino, mono- ou dialkylamino, hydroxy, alcoxy en C1-C4, alkylacyloxy en C1-C4, cycloalkyle en C3-C6 non substitué ou substitué [par alkyle en C1-C4, benzyle non substitué ou substitué par halogène, alkyle en C1-C4, halogénalkyle en C1-C4 ou alcoxy en C1-C4]

sous réserve que lorsque Y est mis pour un groupe C-$R^2$ et $R^1$ par hydrogène, $R^2$ ne représente pas hydrogène, méthyle, trifluorométhyle, phényle, pyridyle et benzyle, lorsque simultanément X est mis pour NH, de même R2 ne représente pas hydrogène, méthyle ou trifluorométhyle
quand simultanément X est mis pour N-benzyle ou N-butyle; et en outre, sous réserve que dans le cas ou X = N$R^3$ et Y = N, les restes $R^1$ et $R^3$ ne représentent pas simultanément l'hydrogène.

**2.** Dérivés de quinoléine condénsés hétérocycliques à noyau pentagonal de formule I selon la revendication 1 dans laquelle $R^1$ est mis pour alkyle en C1-C4 substitué par halogène ou pour halogène et Y est mis pour C-$R^2$

**3.** Procédé de préparation de 3H-imidazol [4,5-h]quinoléine de formule I selon la revendication 1, dans laquelle X est mis pour N-$R^3$ et Y pour C-$R^2$, caractérisé par le fait que l'on fait réagir de la 7,8-diamino-quinoléine de formule II

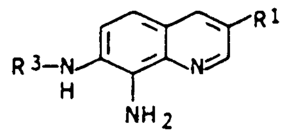

(II)

de manière connue en soi, avec de l'acide carboxylique de formule R2-COOH (III) ou de l'orthoester de formule $R^2C(OR^4)_3$ (VIV) dans laquelle $R^4$ représente alkyle en C1-C4.

**4.** Procédé de préparation d'oxazolo [5,4-h]quinoléine de formule I selon la revendication 1, dans laquelle X est mis pour l'oxygène et Y pour C-$R^2$, caractérisé par le fait que l'on fait réagir de la 7-hydroxy-8-aminoquinoléine de formule V

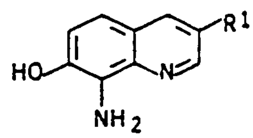

(V)

de manière connue en soi, avec de l'acide carboxylique de formule $R^2$-COOH(III) ou orthoester de formule $R^2$-$C(OR^4)_3$ (IV) dans laquelle $R^4$ représente alkyle en C1-C4.

**5.** Procédé de préparation de triazolo [5,4-h]quinoléine de formule I selon la revendication 1 dans laquelle X est mis pour N-$R^3$ et Y pour l'azote, caractérisé par le fait que l'on cyclise de la 7,8-diaminoquinoléine de formule VI

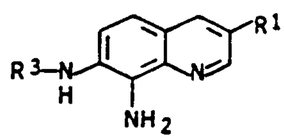

(VI)

de manière connue en soi, par diazotation avec du nitrite de sodium en solution d'acide chlorhydrique.

**6.** Herbicide, contenant des additifs inertes et un dérivé de quinoléine condensé hétérocyclique à noyau pentagonal de formule I selon la revendication 1.

**7.** Procédé de lutte contre la croissance des plantes indésirables, caractérisé par le fait que l'on traite les plantes et/ou leur biotope avec une quantité à effet herbicide d'un dérivé de quinoléine condensé hétérocyclique à noyau pentagonal de formule I

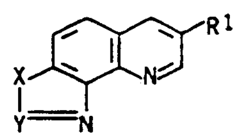

(I)

dans laquelle $R^1$, Y et X ont les significations suivantes :

- $R^1$ hydrogène, halogène, carboxyle ou alkyle en C1-C6 éventuellement substitué par alcoxy en C1-C4, alkylthio en C1-C4 ou halogène
- Y azote ou un groupe C-$R^2$, dans lequel
- $R^2$ représente hydrogène, halogène, hydroxy, alcoxy en C1-C4, alkylthio en C1-C4, un reste alkyle en C1-C6 non substitué ou substitué par halogène, hydroxy, alcoxy, cyano, alcoxy en C1-C4 ou alkylthio en C1-C4, un reste cycloalkyle en C3-C6 non substitué ou substitué par alkyle en C1-C4, un reste benzyle, phéthyle, phényle ou naphtyle non substitué ou substitué par nitro, amino, halogène, alkyle en C1-C4, alcoxy en C1-C4, alkyle en C1-C4-thio, halogénalkyle en C1-C4, acylamino ou acyloxy, ou un noyau hétérocyclique à 5 ou 6 chaînons, avec

un ou deux hétéroatomes choisis dans le groupe oxygène soufre et azote, noyau éventuellement substitué par nitro, alkyle en C1-C4 ou halogène

X oxygène ou le reste N-$R^3$, $R^3$ représentant hydrogène, alkyle en C1-C6, alcényle en C2-C6, alcinyle en C2-C6 non substitué ou substitué par halogène, amino, mono- ou dialkylamino, hydroxy, alcoxy en C1-C4, alkylacyloxy en C1-C4, cycloalkyle en C3-C6 non substitué ou substitué par alkyle en C1-C4, benzyle non substitué ou substitué par halogène, alkyle en C1-C4, halogénalkyle en C1-C4 ou alcoxy en C1-C4.